(19) Europäisches Patentamt
European Patent Office
Office européen des brevets

(11) Veröffentlichungsnummer : 0 279 162 B1

# (12) EUROPÄISCHE PATENTSCHRIFT

(45) Veröffentlichungstag der Patentschrift : 19.06.91 Patentblatt 91/25

(21) Anmeldenummer : 88100093.9

(22) Anmeldetag : 07.01.88

(51) Int. Cl.[5] : C07C 317/00, C07C 321/00, C07C 323/02, C07C 323/30, C07C 59/125, C07C 69/708, C07C 233/00, A61K 31/19, A61K 31/215

## (54) Neue Carbonsäurederivate, Verfahren zu ihrer Herstellung sowie Arzneimittel, die diese Verbindungen enthalten.

(30) Priorität : 13.01.87 DE 3700729

(43) Veröffentlichungstag der Anmeldung : 24.08.88 Patentblatt 88/34

(45) Bekanntmachung des Hinweises auf die Patenterteilung : 19.06.91 Patentblatt 91/25

(84) Benannte Vertragsstaaten : AT BE CH DE ES FR GB GR IT LI LU NL SE

(56) Entgegenhaltungen :
EP-A- 0 190 682
DE-A- 3 525 284
GB-A- 1 382 267
GB-A- 2 090 591
US-A- 3 562 330
TETRAHEDRON LETTERS, Bd. 23, Nr. 33, 1982, Pergamon Press Ltd.; D.J. AGER: "The ester enolate claisen rearrangement of allyl alpha-hydrocyacetates and alpha-phenylthioacetates."
TETRAHEDRON LETTERS, Nr. 48, 1977, Pergamon Press Ltd.; B. LYTHGOE et al.: "A new 1,2-elimination reaction with a radical mechanism"

(56) Entgegenhaltungen :
JOURNAL OF THE AMERICAN CHEMICAL SOCIETY, Bd. 105, Nr. 18, 07.09.1983; J.G. HEXEM et al.: "C NMR of Crystalline Morphine"
JOURNAL OF THE AMERICAN CHEMICAL SOCIETY, Bd. 105, Nr. 26, 28.12.1983, B. LAM et al.: "Polarized Nonvertical Excited States: FORS MCSCF and CI study of Torsion and Bending in Allene"
CHEMICAL ABSTRACTS, Bd. 90, Nr. 7, 12.02.1979, Seite 107, Referat Nr. 49008f, Columbus, Ohio, USA; K. PRASAD et al.: "Potential antitubercular drugs. Part I. Synthesis of derivatives of myristic and stearic acids."

(73) Patentinhaber : BOEHRINGER MANNHEIM GMBH
Sandhofer Strasse 116
W-6800 Mannheim 31 (DE)

(72) Erfinder : Wolff, Hans Peter, Dr.rer.nat
Rebenweg 24
W-6945 Hirschberg-Grosssachsen (DE)
Erfinder : Witte, Ernst-Christian, Dr.rer.nat
Beethovenstrasse 2
W-6800 Mannheim 1 (DE)
Erfinder : Kühnle, Hans-Frieder, Dr.rer.nat
Silcher Weg 6
W-6940 Weinheim (DE)



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind neue Carbonsäurederivate der allgemeinen Formel I

```
R1-A-CH-COOH            (I),
     |
   Y-B-R2
```

in welcher

$R_1$ einen $C_1$-$C_8$-Alkoxy-, $C_1$-$C_8$-Alkylthio-, $C_1$-$C_8$-Alkylsulfinyl-, $C_1$-$C_8$-Alkylsulfonyl- oder $C_1$-$C_8$-Alkylaminorest, einen $C_3$-$C_{10}$-Cycloalkyl- oder $C_3$-$C_{10}$-Cycloalkoxyrest, einen Phenyl-, Phenyloxy-, Phenylthio-, Phenylsulfinyl-, Phenylsulfonyl- oder Phenylaminogruppe, wobei die Phenylteile der zuvor genannten Reste ein- oder mehrfach durch Hydroxy-, Halogen-, $C_1$-$C_6$-Alkyl-, $C_1$-$C_6$-Alkoxy-, Trifluormethyl-, Cyano-, Nitro-, Amino-, $C_l$-$C_6$-Alkylamino- oder Di-$C_l$-$C_6$-alkylaminogruppen substituiert sein können,

$R_2$ einen Phenylrest, der ein- oder mehrfach durch Halogen-, $C_1$-$C_6$-Alkyl-, $C_1$-$C_6$-Alkoxy und Trifluormethylgruppen substituiert sein kann, oder für den Fall, daß B eine Alkylengruppe ist, auch Wasserstoff,

A die Gruppen $-(CH_2)_o-$ mit o = 3-10 oder $-(CH_2)p$-X-$(CH_2)q$ mit p = 2-8 und q = 1-6, wobei o, p, q ganze Zahlen bedeuten und die Summe von p und q nicht größer als 10 sein darf und X ein Sauerstoff- oder Schwefelatom oder die Gruppe –NH– bedeutet, oder, falls $R_1$ einen wie in der Definition von $R_1$ angegebenen Phenylrest darstellt, A auch die Gruppen $-CH_2$-X-$(CH_2)q$, $-CH{=}CH-CH_2-$, $-C{\equiv}C-CH_2-$, $-C{\equiv}C-(CH_2)p-$, $-CH{=}CH-CH_2$-X-$(CH_2)q$, $-C{=}C-CH_2$-X-$(CH_2)q$, wobei p, q und X die oben angegebene Definition besitzen,

Y die Gruppe $-S(O)_n-$ oder –O– mit n = 0, 1 oder 2 und

B einen Valenzstrich oder die Gruppe $-(CH_2)_r-$ mit r = 1-18, $-CH_2-CH{=}CH-$, $-CH_2-C{\equiv}C-$ oder $-CH{=}CH-$ bedeuten mit Ausnahme der Verbindungen 2-Phenoxy-5-phenylvaleriansäure, 2-Phenylthio-5-phenyl-4-pentensäure und 2-Phenylsulfonyl-5-phenyl-4-pentensäure, sowie deren physiologisch unbedenklichen Salze, Ester und Amide, Verfahren zu ihrer Herstellung sowie Arzneimittel, die diese Verbindungen enthalten.

Von den Verbindungen der allgemeinen Formel 1 sind bisher nur wenige Beispiele bekannt. Die erfindunsgemäße pharmakologische Wirkung ist hierbei noch nicht beschrieben worden :

2-Phenoxy-5-phenylpentansäure wird von Nordin, US-Patent 3.562.330, als Vorstufe von antiarrhythmisch wirksamen Aminen beschrieben.

5-Phenyl-2-phenylsulfonyl-4-pentansäure-methylester wird von Trost und Hung, J. Am. Chem. Soc. 1983, 105, 7757, als Zwischenprodukt beschrieben.

2-Methyl-2-methylthio-5-phenylpentansäure und phenylsubstituierte Analoga sowie 2-Methyl-2-methylthio-6-(3-methoxyphenyl)hexansäure und 2-Methyl-2-methylthio-7-(3,4-methylen-dioxiphenyl)heptansäure werden von Trost et al., J. Org. Chem. 1978, 43, 4549, als Zwischenprodukte zur Herstellung von Enolthioethern verwendet. Ferner ist in Tetrahedron Letters, 46, 1977, 4223 - 4225 die Verbindung 2-Phenylthio-5-phenyl-4-pentensäure vorbeschrieben, und aus US-A-3,562,330 sind die beiden Verbindungen 2-Phenoxy-5-phenyl-valeriansäure und deren Amid vorbekannt. Außerdem ist die Verbindung 2-Phenylsulfonyl-5-phenyl-4-pentensäure aus J. Am. Chem. Soc. 105, 1983, 7757 – 7759 literaturbekannt. Diese Verbindungen werden durch den oben angegebenen Disclaimer von der vorliegenden Erfindung nicht umfaßt.

Strukturell ähnliche Carbonsäurederivate zu Verbindungen der Formel I werden in der früheren PCT-Anmeldung WO-A-8700521 beschrieben.

Die Verbindungen der allgemeinen Formel I besitzen wertvolle pharmakologische Eigenschaften. Sie sind als Arzneimittel zur Behandlung von Diabetes, Praediabetes und insbesondere zur Behandlung von Altersdiabetes geeignet.

Die Verbindungen der Formel I haben zu bekannten Antidiabetica strukturell und in der Wirkungsart keine Beziehung. Die senken den Blutzuckerspiegel durch Verstärkung der peripheren Glucoseoxidation. Ihre Wirkung beruht auf einer Erhöhung der Sensitivität von peripherem Gewebe gegenüber Insulin. Im Gegensatz zu den Biguaniden wird dabei kein Anstieg der Blutlaktatwerte beobachtet. Die Verbindung der allgemeinen Formel I stellen daher auch eine wertvolle Bereicherung bei der Behandlung nicht-diabetischer Krankheitszustände dar, bei denen eine Insulinresistenz vorhanden ist, wie z.B. Adipositas und Atherosklerose.

Zusätzlich zeigen sie eine ausgeprägte lipidsenkende Wirkung und eignen sich daher auch zur Behandlung von Fettstoffwechselerkrankungen.

Unter "Alkyl" resten des Substituenten $R_1$ werden gesättigte oder ungesättigte, geradkettige oder verzweigte aliphatische Kohlenwasserstoffreste mit 1 bis 8 Kohlenstoffatomen verstanden, insbesondere der Methyl-, t-Butyl, n-Hexyl- und n-Octylrest.

Der Begriff Cycloalkyl- oder Cycloalkoxyreste soll für gesättigte carbocyclische Ringe mit 3 bis 10 Kohlenstoffatomen stehe, die gegebenenfalls bi- und tricyclische Systeme bilden und Substituenten wie Halogen oder niedere Alkylgruppen tragen. Insbesondere bevorzugt ist der Cyclopentyl-, der Cyclohexyl- und der 1-Methylcyclohexylrest bzw. der Cyclohexyloxy- und 1-Methylcyclohexyloxy-Rest.

Unter Arylresten werden aromatische Kohlenwasserstoffe mit 6 bis 14 Kohlenstoffatomen verstanden, bevorzugt die Phenyl- und Naphthylgruppe.

Unter substituierten Arylresten werden in allen Definitionen solche aromatische Kohlenwasserstoffe mit 6 bis 14 Kohlenstoffatomen verstanden, die in einer oder mehreren Positionen die Hydroxylgruppe, Halogen, niedere Alkyl-, niedere Alkoxy-, Trifluormethyl-, Cyano-, Nitro- oder gegebenenfalls ein- oder zweifach durch niederes Alkyl substituierte Aminogruppe tragen. Insbesondere kommen dafür gegebenenfalls durch die vorgenannten Gruppen substituierte Phenyl- oder Naphthylreste infrage. Besonders bevorzugt ist der Phenyl-, 4-Methylphenyl-, 4-t-Butylphenyl-, 4-Methoxyphenyl-, 3-Trifluormethylphenyl- und der 4-Chlorphenylrest. Dabei versteht man unter Halogen Fluor, Chlor, Brom und Jod, vorzugsweise Fluor, Chlor und Brom.

Niedere Alkyl- bzw. Alkoxy-Reste können 1 – 6 C-Atome aufweisen, bevorzugt sind der Methyl- und Ethylsowie der Methoxy- und Ethoxy-Rest.

Für unverzweigte Alkylengruppen A kommen insbesondere die folgenden infrage :

a) $-(CH_2)_o-$ mit o = 3-10 und

$-(CH_2)_p-X-(CH_2)_q-$ mit p = 2 – 8 und q = 1-6,

wobei o, p and q ganze Zahlen bedeuten, die Summe von p und q nicht größer als 10 sein darf und X ein Sauerstoff- oder Schwefelatom oder die Gruppe NH bedeutet.

b) Falls $R_1$ einen Arylrest darstellt, kann A auch eine der nachstehenden Gruppen bedeuten :

$-CH_2-X-(CH_2)q-$ ,

$-C{=}CHCH_2-$ , $-C{\equiv}CCH_2-$ , $-C{\equiv}C(CH_2)p-$ ,

$-CH{=}CHCH_2-X-(CH_2)q-$ , $-C{\equiv}CCH_2-X-(CH_2)q$,

worin p, q und X die oben angegebenen Bedeutungen besitzen.

Als verzweigte Alkylengruppen kommen zum Beispiel in Betracht :

a)

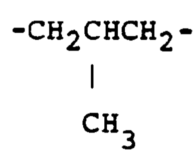

und

b) für den Fall, daß $R_1$ einen Arylrest darstellt, auch die Gruppen

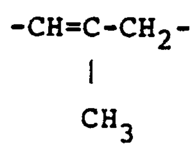 und 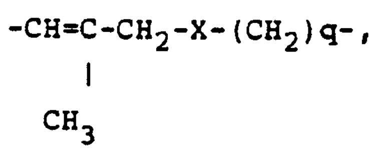,

worin X und q die oben angegebenen Bedeutungen besitzen.

Unter einer Alkylengruppe B sind insbesondere die Reste

$-(CH_2)r-$ , $-CH_2-CH{=}CH-$ , $-CH_2C{\equiv}C-$ und $-CH{=}CH-$

zu verstehen, worin r eine ganze Zahl von 1 bis 18 bedeutet.

Als physiologisch unbedenkliche Salze kommen insbesondere Alkali-, Erdalkali- oder Ammoniumsalze (sowie gegebenenfalls Salz mit blutzuckersenkenden Biguaniden) infrage.

Die von den Carbonsäuren der allgemeinen Formel I abgeleiteten Ester enthalten als Alkoholkomponente niedere einwertige Alkohole, von denen Methanol, Ethanol und n-Butanol bevorzugt sind, sowie mehrwertige Alkohole wie z.B. Glycerin, oder Alkohole mit anderen funktionellen Gruppen, wie z.B. Ethanolamin.

Die erfindungsgemäßen, von den Carbonsäuren der allgemeinen Formel I abgeleiteten Amide enthalten als Aminkomponente vorzugsweise Ammoniak, p-Aminobenzoesäure, β-Alanin, Ethanolamin oder 2-Aminopropanol. Es kommen aber auch Alkylamine wie z.B. Isopropylamin oder tert.-Butylamin, Dialkylamine wie

Diethylamin sowie cyclische Amine wie z.B. Morpholin oder 4-substituierte Piperazine in Frage.

Die substituierten Carbonsäuren der allgemeinen Formel I besitzen ein Chiralitätszentrum. Die obige Definition der erfindungsgemäßen Verbindungen schließt daher auch alle möglichen Enantiomeren, ihre Gemische und die Racemate ein.

Insbesondere bevorzugt sind Carbonsäurederivate der Formel I, in denen

$R_1$ einen Phenyl- oder Phenyloxyrest bedeutet, der gegebenenfalls ein- oder mehrfach durch Halogen-, $C_1$-$C_6$-Alkyl-, $C_1$-$C_6$-Alkoxy- und Trifluormethylgruppen substituiert sein kann,

$R_2$ einen Phenyl-, 2-Methylphenyl-, 3-Methylphenyl-, 4-Methylphenyl, 4-tert.-Butylphenyl-, 4-Methoxy- und 3-Trifluormethylphenylrest,

A die Gruppe $-(CH_2)_o-$ mit o = 4, 5 oder 6,

Y die Gruppe $S(O)_n$ oder O,

n die Zahlen 0, 1 oder 2 und

B ein Valenzstrich oder eine geradkettige, gesättigte oder ungesättigte Alkylengruppe mit 1 bis 5 Kohlenstoffatomen

bedeutet,
sowie deren physiologisch unbedenklichen Salze, Ester und Amide.

Gegenstand der Erfindung sind weiterhin Verfahren zur Herstellung der Verbindungen der allgemeinen Formel I, in dem man

A. eine Gruppe der allgemeinen Formel II

```
Z-CH-W          (II),
  |
  X
```

in welcher

W $-COOR_3$ oder eine andere in die Carboxylfunktion überführbare Gruppe

X ein reaktiver Rest und

Z Wasserstoff oder die Gruppe $R_1$-A–, in welcher $R_1$ und A die oben angegebenen Bedeutungen besitzen und

$R_3$ niederes Alkyl

bedeutet,
mit einer Gruppe der allgemeinen Formel III

$$R_2\text{-B-YH}, \quad \text{(III)}$$

in welcher $R_2$, B und Y die oben angegebenen Bedeutungen besitzen, zu einer Verbindung der allgemeinen Formel IV

```
Z-CH-W          (IV)
  |
  Y-B-R2
```

umsetzt und im Anschluß daran gegebenenfalls

a) die Gruppe W in die freie Carboxylfunktion, einen physiologisch verträglichen Ester oder ein Amid umwandelt,

b) für den Fall, daß Y ein Schwefelatom darstellt, die Gruppe Y in die Gruppe SO oder $SO_2$ umwandelt oder

c) für den Fall, daß Z Wasserstoff bedeutet, entweder

C1) mit einer Verbindung der allgemeinen Formel V

$$R_1\text{-A-X}, \quad \text{(V)}$$

in welcher $R_1$ und A die oben angegebenen Bedeutungen besitzen und X eine reaktive Gruppe bedeutet,

alkyliert oder
C2) mit einer Verbindung der allgemeinen Formel VI

$$R_1\text{-}A'\text{-}CHO, \quad (V')$$

in welcher A' einen um die $CH_2$-Gruppe verkürzten Alkylenrest A bedeutet und
$R_1$ die oben angegebene Bedeutung besitzt, kondensiert und im Anschluß an die Kondensation die entstandene Doppelbindung hydriert und die aus den vorgenannten Verfahrensschritten C1) oder C2) erhaltenen Verbindungen IV mit Z = $R_1$A– in der oben angegebenen Weise an den Gruppen W oder Y umwandelt oder
B. eine Verbindung der allgemeinen Formel VI

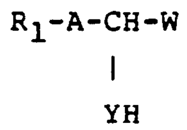

(VI),

in welcher $R_1$, A, Y und W die oben angegebenen Bedeutungen besitzen, mit einer Gruppe der allgemeinen Formel VII

$$R_2\text{-}B\text{-}X \quad (VII)$$

in welcher $R_2$, B und X die oben angegebenen Bedeutungen besitzen,
zu einer Verbindung der Formel IV umsetzt und diese gegebenenfalls in der oben angegebenen Weise an den Gruppen W und Y umwandelt.

Die Herstellung der Ausgangsverbindungen kann nach an sich bekannten Methoden erfolgen, in dem man z.B. Malonester mit Verbindungen der Formel V alkyliert und die erhaltenen Derivate der Formel VIII

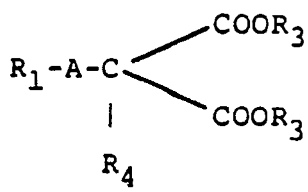

(VIII),

in welcher $R_1$, A und $R_3$ die oben angegebenen Bedeutungen besitzen und $R_4$ Wasserstoff bedeutet,
zu Verbindungen VIII umsetzt, in welcher $R_4$ eine reaktive Gruppe X bedeutet, diese anschließend durch Decarboxylierung in die Verbindungen der allgemeinen Formel II, in welcher W die Gruppe $-COOR_3$ und Z die Gruppe $R_1$-A– bedeutet, umwandelt und diese dann gegebenenfalls in Verbindungen der allgemeinen Formel VI überführt.

Die Umsetzung der reaktiven Carbonsäurederivate der allgemeinen Formel II mit den Verbindungen der allgemeinen Formel III erfolgt zweckmäßig unter Zusatz eines säurebindenden Mittels wie z.B. Natriumhydrogencarbonat, Kaliumcarbonat, Natriumethylat oder Natriumhydrid. Vorzugsweise werden für die Umsetzung Ester der reaktiven Carbonsäurederivate verwendet. Als inertes Lösungsmitel gelangen z.B. Ether, Benzol, Tetrahydrofuran, Dioxan oder Methylenchlorid zum Einsatz. Bei Verwendung anorganischer Basen verwendet man als Reaktionsmedium auch z.B. Ethanol, Butanon-2, Dimethylformamid, Hexamethylphosphorsäuretriamid oder Acetontril. Als reaktiver Rest X kommen hier z.B. Halogenide- oder Sulfonsäureestergruppen infrage, insbesondere Chlorid, Bromid, p-Toluolsulfonyloxy und Methansulfonyloxy.

Die Umsetzung der Verbindungen IV, Z = Wasserstoff, mit Aldehyden der Formel VI nach Verfahren C2) findet unter Bedingungen statt, wie sie für die Kondensation aktivierter Methylengruppen mit Ketoverbindungen üblich sind. Vorzugsweise wird die Kondensation in Pyridin oder Dimethylformamid unter Zusatz katalytischer Mengen einer starken Base wie z.B. Piperidin durchgeführt. Vorteilhaft setzt man dem Reaktionsgemisch ein geeignetes Lösungsmittel wie z.B. Benzol zu, um das Reaktionswasser azeotrop abzudestillieren.

Die nachfolgende Hydrierung der entstandenen Doppelbindung wird in üblicher Weise mit katalytisch erregtem Wasserstoff bei Normaldruck oder bei erhöhtem Druck durchgeführt. Als Katalysatoren kommen Metallkatalysatoren wie z.B. Raney-Nickel oder Palladium-Kohle in Betracht. Als Lösungsmittel sind z.B. Essigsäure oder niedere Alkohole, im Falle von Carbonsäuren IV auch wässriges Alkali geeignet.

Die Umsetzung der reaktiven Derivate V mit Verbindungen der allgemeinen Formel IV mit Z = Wasserstoff und der reaktiven Derivate VII mit Verbindungen der allgemeinen Formel VI erfolgt zweckmäßig unter Zusatz einer starken Base wie z.B. Natriumethylat, Natriumhydrid oder 1,8-Diazabicylo-(5, 4, 0)undec-7-en. Als inertes Lösungsmittel dienen z.B. Ethanol, Dimethylsulfoxid, Toluol oder Benzol. Weiterhin kommen z.B. Dimethylformamid oder Hexamethylphosphorsäuretriamid als Lösungsmittel in Betracht. Die Reaktion wird vorzugsweise bei Zimmertemperatur oder mäßig erhöhter Temperatur oder bei der Siedetemperatur des verwendeten Lösungsmittels durchgeführt. Als reaktiver Rest X kommen hier z.B. Halogenide- oder Sulfonsäureestergruppen infrage, insbesondere Chlorid, Bromid, p-Toluolsulfonyloxy und Methansulfonyloxy.

Die Oxidation von Verbindungen der allgemeinen Formel IV, in welchen Y S bedeutet, zu Sulfoxiden oder Sulfonen wird vorzugsweise mit Wasserstoffperoxid in polaren Lösungsmitteln wie Eisessig, einem Gemisch von Eisessig und Acetanhydrid oder Aceton durchgeführt. Besonders vorteilhaft hat sich die Oxidation mit Trifluorperessigsäure erwiesen. Als Lösungsmittel wird hierbei zweckmäßig Trifluoressigsäure verwendet.

Unter der in eine Carboxylfunktion überführbare Gruppe W soll insbesondere die Nitrilgruppe oder ein Rest verstanden werden, der sich oxidativ in die Carboxylfunktion überführen läßt. Als oxidierbare Gruppen kommen vorzugsweise die Hydroxymethyl-, die Aminomethyl- und die Formylgruppe oder funktionelle Derivate dieser Gruppen infrage. Die Oxidation läßt sich mit den üblichen Oxidationsmitteln wie z.B. Mangan-IV-Verbindungen, Permanganaten, Dichromaten, im Falle der Formylgruppe auch mit Luftsauerstoff und Silberoxid durchführen.

Die gegebenenfalls im Anschluß an die Kondensation zu den Verbindungen der allgemeinen Formel IV durchzuführende Umwandlung des Substituenten W erfolgt beispielsweise durch Verseifen der Carbonsäureester zu den entsprechenden Carbonsäure mit Mineralsäuren oder Alkalihydroxiden in einem polaren Lösungsmittel (wie Wasser, Methanol, Ethanol, Dioxan oder Aceton). Vorteilhaft wird die Verseifung mit einer starken Base (wie Natrium- oder Kaliumhydroxid) in einem Gemisch aus Methanol und Wasser bei Raumtemperatur oder bei mäßig erhöhten Temperaturen durchgeführt. Umgekehrt kann man aber auch die Carbonsäuren in üblicher Weise verestern oder Ester mit einem bestimmten Rest $R_4$ durch Umestern in einen Ester mit einem anderen Rest $R_4$ umwandeln. Die Veresterung der Carbonsäuren wird zweckmäßig in Gegenwart eines sauren Katalysators, wie z.B. Chlorwasserstoff, Schwefelsäure, p-Toluolsulfonsäure oder eines stark sauren Ionenaustauschharzes, vorgenommen.

Umesterungen hingegen erfordern den Zusatz einer geringen Menge einer basischen Substanz, z.B. eines Alkali- oder Erdalkalihydroxids oder eines Alkalialkoholats. Für die Veresterung der Carboxylgruppe bzw. für eine Umesterung eignen sich prinzipiell alle Alkohole. Bevorzugt sind die niederen einwertigen Alkohole wie Methanol, Ethanol oder Propanol sowie mehrwertige Alkohole, z.B. Glycerin, oder Alkohole mit anderen funktionellen Gruppen, wie z.B. Ethanolamin.

Die erfindungsgemäßen, von den Carbonsäuren der allgemeinen Formel I abgeleiteten Amide werden bevorzugt nach an sich bekannten Methoden aus den Carbonsäuren oder ihren reaktiven Derivaten (wie z.B. Carbonsäurehalogeniden, -estern, -aziden, -anhydriden oder gemischten Anhydriden) durch Umsetzung mit Aminen hergestellt. Als Aminkomponenten kommen z.B. Ammoniak, Alkylamine, Dialkylamine, aber auch Aminoalkohole wie z.B. Ethanolamin und 2-Aminopropranol sowie Aminosäuren wie z.B. p-Aminobenzoesäure, β-Alanin und andere infrage. Andere wertvolle Aminkomponenten sind Alkyl-, Aralkyl- und Arylpiperazine.

Die Herstellung der obigen Amide kann aber auch durch partielle Verseifung der von den erfindungsgemäßen Carbonsäuren abgeleiteten Nitrilen erfolgen. Die Verseifung erfolgt in verdünnten Mineralsäuren bei mäßig erhöhten Temperaturen, in alkalischer Hydroperoxidlösung oder vorteilhaft in 98 proz. Schwefelsäure oder Polyphosphorsäure.

Zur Herstellung von Salzen mit pharmakologisch verträglichen organischen oder anorganischen Basen, wie z.B. Natriumhydroxid, Kaliumhydroxid, Calciumhydroxid, Ammoniumhydroxid, Methylglukamin, Morpholin oder Ethanolamin können die Carbonsäuren mit den entsprechenden Basen umgesetzt werden. Auch Mischungen der Carbonsäure mit einem geeigneten Alkalicarbonat bzw. -hydrogencarbonat kommen in Betracht.

Zur Herstellung von Arzneimitteln werden die Verbindungen der allgemeinen Formel In in an sich bekannter Weise mit geeigneten pharmazeutischen Trägersubstanzen, Aroma-, Geschmacks- und Farbstoffen gemischt und beispielsweise als Tabletten oder Dragées ausgeformt oder unter Zugabe entsprechender Hilfsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure),

hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0,1 bis 50 mg/kg Körpergewicht. Normalerwise sind 0,5 bis 40 und vorzugsweise 1,0 bis 20 mg/kg/Tag in einer oder mehreren Anwendungen pro Tag wirksam, um die gewünschten Resultate zu erhalten.

Bevorzugt im Sinne der Erfindung sind außer den in den Beispielen genannten Verbindungen der Formel I die folgenden Verbindungen sowie deren Amide, Methyl- und Ethylester :

8-Methoxy-2-(4-methylphenoxy)octansäure
8-Methoxy-2-(4-methylphenylthio)octansäure
8-Methoxy-2-(2-phenylethylsulfonyl)octansäure
8-Methylthio-2-(4-methylphenoxy)octansäure
8-Methylthio-2-(4-methylphenylthio)octansäure
8-Methylamino-2-(4-methylphenylsulfonyl)octansäure
4-Pentyloxy-2-(4-methylphenylsulfonyl)buttersäure
6-Cyclopentyl-2-(4-methylphenylsulfonyl)hexansäure
6-Cyclohexyl-2-(4-methylphenylsulfonyl)hexansäure
6-Cyclohexyloxy-2-(4-methylphenylsulfonyl)hexansäure
6-(1-Methylcyclohexyloxy)-2-(4-methylphenylsulfonyl)hexansäure
8-Cyclohexyl-2-(4-methylphenylsulfonyl)octansäure
5-(4-Methylphenyl)-2-(4-methylphenylsulfonyl)-4-pentinsäure : Fp : 134-135°C ; Ethylester : Fp : 65°C
5-(4-t-Butylphenyl)-2-(4-methylphenylsulfonyl)-4-pentinsäure : farbloses Öl
5-(4-Chlorphenyl)-2-t-butoxy-4-pentinsäure
5-(4-Chlorphenyl)-2-phenoxy-4-pentinsäure
5-(4-Chlorphenyl)-2-(4-methylphenoxy)-4-pentinsäure
5-(4-Chlorphenyl)-2-(4-t-butylphenoxy)-4-pentinsäure
5-(4-Chlorphenyl)-2-(4-chlorphenoxy)-4-pentinsäure
5-(4-Chlorphenyl)-2-(4-methoxyphenoxy)-4-pentinsäure
5-(4-Chlorphenyl)-2-(2-phenylethoxy)-4-pentinsäure
5-(4-Chlorphenyl)-2-(phenylthio)-4-pentinsäure
5-(4-Chlorphenyl)-2-(4-methylthio)-4-pentinsäure : Fp : 110-112°C
5-(4-Chlorpehnyl)-2-(4-t-butylphenylthio)-4-pentinsäure
5-(4-Chlorphenyl)-2-(4-chlorphenylthio)-4-pentinsäure
5-(4-Chlorphenyl)-2-(4-methoxyphenylthio)-4-pentinsäure
5-(4-Chlorphenyl)-2-(2-phenylethylthio)-4-pentinsäure
5-(4-Chlorphenyl)-2-(4-t-butylphenylsulfonyl)-4-pentinsäure
5-(4-Chlorphenyl)-2-(2-phenylethenylsulfonyl)-4-pentinsäure
5-(4-Chlorphenyl)-2-(2-phenylethylsulfonyl)-4-pentinsäure
5-(4-Chlorphenyl)-2-(4-chlorcinnamylsulfonyl)-4-pentinsäure
7-(4-Chlorphenyl)-2-(4-methylphenylsulfonyl)-6-heptinsäure
5-(4-Methoxyphenyl)-2-(4-methylphenoxy)-4-pentinsäure
5-(4-Methoxyphenyl)-2-(4-methylphenylthio)-4-pentinsäure
7-Phenyl-2-(2-phenylethoxy) heptansäure
7-Phenyl-2-(2-phenylethylthio) heptansäure
7-Phenyl-2-(benzylsulfonyl) heptansäure : Öl
7-Phenyl-2-[2-(4-methylphenyl)ethylsulfonyl]heptansäure Fp. 100-101°C
7-Phenyl-2-[2-(4-t-butylphenyl)ethylsulfonyl]heptansäure Fp. 88°C
7-Phenyl-2-[2-(4-chlorphenyl)ethylsulfonyl]heptansäure Fp. 82-84°C
7-Phenyl-2-[2-(4-methoxyphenyl)ethylsulfonyl]heptansäure Fp. 101°C
7-Phenyl-2-(2-phenylethenylsulfonyl)heptansäure Na-Salz ; amorph
7-Phenyl-2-(3-phenylpropylsulfonyl)heptansäure : Öl
7-Phenyl-2-(4-chlorcinnamylsulfonyl)heptansäure
8-Phenyl-2-(4-t-butylphenoxy)octansäure : Fp : 51-53°C

8-Phenyl-2-(4-t-butylphenylthio)octansäure
8-Phenyl-2-(4-ethylphenylsulfonyl)octansäure : Fp : 72-73°C
8-Phenyl-2-(4-propylphenylsulfonyl)octansäure : Fp : 75-76°C
8-Phenyl-2-(4-t-butylphenylsulfonyl)octansäure
8-Phenyl-2-(2-phenylethylsulfonyl)octansäure : Fp : 131-133°C
7-(4-Chlorphenyl)-2-(4-t-butylphenoxy)heptansäure : Öl
7-(4-Chlorphenyl)-2-(2-phenylethoxy)heptansäure
7-(4-Chlorphenyl)-2-(2-methylphenylthio)heptansäure : Fp : 55-56°C
7-(4-Chlorphenyl)-2-(4-t-butylphenylthio)heptansäure Na-Salz ; amorph
7-(4-Chlorphenyl)-2-(2-phenylethylthio)heptansäure : Fp : 58-60°C
7-(4-Chlorphenyl)-2-(ethylsulfonyl)heptansäure
7-(4-Chlorphenyl)-2-(butylsulfonyl)heptansäure
7-(4-Chlorphenyl)-2-(t-butylsulfonyl)heptansäure
7-(4-Chlorphenyl)-2-(octylsulfonyl)heptansäure Fp. 49°C
7-(4-Chlorphenyl)-2-(4-t-butylphenylsulfonyl)heptansäure Na-Salz ; Fp. 14
7-(4-Chlorphenyl)-2-(2-phenylethylsulfonyl)heptansäure : Fp : 54-56°C
7-(4-Chlorphenyl)-2-(6-phenylhexylsulfonyl)heptansäure
8-(4-Chlorphenyl)-2-(4-t-butylphenoxy)octansäure
8-(4-Chlorphenyl)-2-(2-phenylethoxy)octansäure
8-(4-Chlorphenyl)-2-(4-t-butylphenylthio)octansäure
8-(4-Chlorphenyl)-2-(2-phenylethylthio)octansäure
8-(4-Chlorphenyl)-2-(2-methylphenylsulfonyl)octansäure : Fp : 82-85°C Ethylester : Öl
8-(4-Chlorphenyl)-2-(4-ethylphenylsulfonyl)octansäure
8-(4-Chlorphenyl)-2-(4-prophylphenylsulfonyl)octansäure
8-(4-Chlorphenyl)-2-(4-t-butylphenylsulfonyl)octansäure
8-(4-Chlorphenyl)-2-(2-phenylethylsulfonyl)octansäure
8-(4-Chlorphenyl)-2-[2-(4-methylphenyl)ethylsulfonyl]octansäure
8-(4-Chlorphenyl)-2-[2-(4-chlorphenyl)ethylsulfonyl]octansäure
8-(4-Chlorphenyl)-2-[2-(4-methoxyphenyl)ethylsulfonyl]octansäure
7-(4-Methoxyphenyl)-2-(4-t-butylphenoxy)heptansäure
7-(4-Methoxyphenyl)-2-(4-t-butylphenylthio)heptansäure
7-(4-Methoxyphenyl)-2-(4-t-butylphenylsulfonyl)heptansäure
7-(4-Methoxyphenyl)-2-(2-phenylethylsulfonyl)heptansäure
7-(4-Methoxyphenyl)-2-[2-(4-methylphenyl)ethylsulfonyl]heptansäure
7-(4-Methoxyphenyl)-2-[2-(4-chlorphenyl)ethylsulfonyl]heptansäure
3-(2-Phenylethoxy)-2-(4-methylphenoxy)propionsäure
3-(2-Phenylethoxy)-2-(4-methylphenylsulfonyl)propionsäure
3-(3-Phenylpropoxy)-2-(4-methylphenoxy)propionsäure
3-(3-Phenylpropoxy)-2-(4-methylphenylthio)propionsäure
3-(3-Phenylpropoxy)-2-(4-methylphenylsulfonyl)propionsäure
4-(2-Phenylethoxy)-2-(4-methylphenoxy)buttersäure Na-Salz ; amorph
4-(2-Phenylethoxy)-2-(4-methylphenylthio)buttersäure
4-(2-Phenylethoxy)-2-(4-methylphenylsulfonyl)buttersäure Na-Salz ; Fp > 250°
4-(2-Phenylethoxy)-2-(2-phenylethylsulfonyl)buttersäure
5-Benzyloxy)-2-(4-methylphenoxy)pentansäure
5-Benzyloxy-2-(4-methylphenylsulfonyl)pentansäure
4-Cinnamyloxy-2-(4-methylphenoxy)buttersäure
4-Cinnamyloxy-2-(4-methylphenylthio)buttersäure
4-Cinnamyloxy-2-(4-methylphenylsulfonyl)buttersäure
4-(2-Methyl-3-phenyl-2- propenyloxy)-2-(4-methylphenoxy)buttersäure
4-(2-Methyl-3-phenyl-2- propenyloxy)-2-(methylphenylthio)buttersäure
4-(2-Methyl-3-phenyl-2- propenyloxy)-2-(4-methylphenylsulfonyl)buttersäure
4-[2-(4-Chlorphenyl)ethoxy]-2-methoxybuttersäure
4-[2-(4-Chlorphenyl)ethoxy]-2-hexyloxybuttersäure
4-[2-(4-Chlorphenyl)ethoxy]-2-(methylthio)buttersäure
4-[2-(4-Chlorphenyl)ethoxy]-2-(hexylthio)buttersäure
4-[2-(4-Chlorphenyl)ethoxy]-2-methylsulfonylbuttersäure
4-[2-(4-Chlorphenyl)ethoxy]-2-hexylsulfonylbuttersäure

3-[2-(4-Chlorphenyl)ethoxy]-2-(4-methylphenoxy)propionsäure
3-[2-(4-Chlorphenyl)ethoxy]-2-(4-methylphenylsulfonyl)proprionsäure
3-[3-(4-Chlorphenyl)ethoxy]-2-(4-methylphenoxy)propionsäure
3-[3-(4-Chlorphenyl)ethoxy]-2-(4-methylphenylthio)propionsäure
3-[3-(4-Chlorphenyl)ethoxy]-2-(4-methylphenylsulfonyl)propionsäure
4-[2-(4-Chlorphenyl)ethoxy]-2-(4-methylphenoxy)buttersäure : Fp : 75-76°C
4-[2-(4-Chlorphenyl)ethoxy]-2-(4-methylphenylthio)buttersäure
4-[2-(4-Chlorphenyl)ethoxy]-2-(2-phenylethylsulfonyl)buttersäure
4-[2-(4-Chlorphenyl)ethoxy]-2-[2-(4-methylphenyl)ethylsulfonyl]butteräure
4-[2-(4-Chlorphenyl)ethoxy]-2-(3-trifluormethylphenylsulfonyl)buttersäure
4-[2-(4-Chlorphenyl)ethoxy]-2-(4-methoxyphenylsulfonyl)buttersäure
4-[2-(4-Chlorphenyl)ethoxy]-2-(4-dimethylaminophenylsulfonyl)buttersäure
4-(3-Trifluormethylphenyl)-2-(4-methylphenylsulfonyl)buttersäure
5-(4-Chlorbenzyloxy)-2-(4-methylphenoxy)pentansäure
5-(4-Chlorbenzyloxy)-2-(4-methylphenylthio)pentansäure Na-Salz ; Fp. 255°
5-(4-Chlorbenzyloxy)-2-(4-methylphenylsulfonyl)pentansäure Fp. 111-113°
5-(4-Chlorbenzyloxy)-2-(2-phenylethylsulfonyl)pentansäure
4-(4-Chlorcinnamyloxy)-2-(4-methylphenoxy)buttersäure
4-[3-(4-Chlorphenyl)-2-propinyloxy]-2-(4-methylphenoxy)buttersäure
4-(4-Chlorcinnamyloxy)-2-(4-methtylphenylthio)buttersäure
4-[3-(4-Chlorphenyl)-2-propinyloxy]-2-(4-methylphenylthio)buttersäure
4-(4-Chlorcinnamyloxy)-2-(4-methylphenylsulfonyl)buttersäure
4-[3-(4-Chlorphenyl)-2-propinyloxy]-2-(4-methylphenylsulfonyl)buttersäure
6-Phenoxy-2-(4-methylphenoxy)hexansäure Fp. 93-94°C
6-Phenoxy-2-(4-t-butylphenoxy)hexansäure Fp. 125-126°C
6-Phenoxy-2-(4-methylphenylthio)hexansäure Fp. 73-74°C
6-Phenoxy-2-(4-methylphenylsulfonyl)hexansäure
7-Phenoxy-2-(4-methylphenoxy)heptansäure Fp. 88-89°C
7-Phenoxy-2-(4-methylphenylthio)heptansäure
7-Phenoxy-2-(4-methylphenylsulfonyl)heptansäure
6-Phenoxy-2-(2-phenylethylsulfonyl)hexansäure
6-Phenylthio-2-(4-methylphenylthio)hexansäure Fp. 70-72°C
6-Phenylthio-2-(4-methylphenoxy)hexansäure Fp. 98-100°C
6-Phenylthio-2-(4-methylphenylsulfonyl)hexansäure
6-Phenylsulfonyl-2-(4-methylphenoxy)hexansäure Fp. 105-107°C
6-Phenylsulfonyl-2-(4-methylphenylsulfonyl)hexansäure
6-Phenylamino-2-(4-methylphenylsulfonyl)hexansäure
6-(4-Chlorphenoxy)-2-(4-methylphenoxy)hexansäure : Fp : 93-95°C
7-(4-Chlorphenoxy)-2-(4-methylphenoxy)heptansäure : Fp 102-104°C
6-(4-Chlorphenoxy)-2-(4-t-butylphenoxy)hexansäure : Fp : 108-109°C
6-(4-Chlorphenoxy)-2-(4-Chlorphenoxy)hexansäure Fp. 84-86°C
6-(4-Chlorphenoxy)-2-(4-methylphenylthio)hexansäure : Fp : 92-93°C
6-(4-Chlorphenoxy)-2-(2-phenylethylsulfonyl)hexansäure Fp. 87-88°C
6-(4-Chlorphenylthio)-2-(4-methylphenoxy)hexansäure Fp. 99-101°C
6-(4-Chlorphenylthio)-2-(4-methylphenylsulfonyl)hexansäure
6-(4-Chlorphenylsulfinyl)-2-(4-methylphenoxy)hexansäure
6-(4-Chlorphenylsulfonyl)-2-(4-methylphenoxy)hexansäure Fp. 106-108°C
6-(4-Chlorphenylamino)-2-(4-methylphenoxy)hexansäure
7-(4-Fluorphenoxy)-2-(4-methylphenoxy)heptansäure
7-(4-Fluorphenoxy)-2-(4-methylphenylthio)heptansäure
7-(4-Flurophenoxy)-2-(4-methylphenylsulfonyl)heptansäure
7-(4-Methylphenoxy)-2-(4-methylphenoxy)heptansäure
7-(4-Methylphenoxy)-2-(4-methylphenylthio)heptansäure
7-(4-Methylphenoxy)-2-(4-methylphenylsulfonyl)heptansäure
7-(4-Methoxyphenoxy)-2-(4-methylphenoxy)heptansäure
7-(4-Methoxyphenoxy)-2-(4-methylphenylthio)heptansäure
7-(4-Methoxyphenoxy)-2-(4-methylphenylsulfonyl)heptansäure
7-(3-Trifluormethylphenoxy)-2-L(4-methylphenoxy)heptansäure

7-(3-Trifluormethylphenoxy)-2-(4-methylphenylsulfonyl)heptansäure
4-(2-Phenoxyethoxy)-2-(4-methylphenoxy)buttersäure
4-(2-Phenoxyethoxy)-2-(4-methylphenylthio)buttersäure
4-(2-Phenoxyethoxy)-2-(4-methylphenylsulfonyl)buttersäure
4-[2-(4-Methylphenoxy)ethoxy]-2-(4-methylphenoxy)buttersäure
4-[2-(4-Methylphenoxy)ethoxy]-2-(4-methylphenylthio)buttersäure
4-[2-(4-Methylphenoxy)ethoxy]-2-(4-methylphenylsulfonyl)buttersäure
4-[2-(4-Chlorphenoxy)ethoxy]-2-(4-methylphenoxy)buttersäure
4-[2-(4-Chlorphenoxy)ethoxy]-2-(4-methylphenylthio)buttersäure
4-[2-(4-Chlorphenoxy)ethoxy]-2-(4-methylphenylsulfonyl)buttersäure
4-[2-(4-Chlorphenoxy)ethoxy]-2-(2-phenylethylsulfonyl)buttersäure
4-[2-(4-Chlorphenoxy)ethoxy]-2-(4-chlorcinnamylsulfonyl)buttersäure
4-[2-(4-Methylphenoxy)ethylthio]-2-(4-methylphenoxy)buttersäure
4-[2-(4-Methylphenoxy)ethylthio]-2-(4-methylphenylsulfonyl)buttersäure
4-[2-(4-Chlorphenoxy)ethylthio]-2-(4-methylphenoxy)buttersäure
4-[2-(4-Chlorphenoxy)ethylthio]-2-(4-methylphenylsulfonyl)buttersäure
4-[2-(4-Chlorphenoxy)ethylamino]-2-(4-methylphenoxy)buttersäure
4-[2-(4-Chlorphenoxy)ethylamino]-2-(4-methylphenysulfonyl)buttersäure
4-[2-(Phenylthio)ethoxy]-2-(4-methylphenylsulfonyl)buttersäure
4-[2-(Phenylsulfinyl)ethoxy]-2-(4-methylphenylsulfonyl)buttersäure
4-[2-(Phenylsulfonyl)ethoxy]-2-(4-methylphenylsulfonyl)buttersäure
4-[2-(Phenylamino)ethoxy]-2-(4-methylphenylsulfonyl)buttersäure
8-(4-Chlorphenoxy)-2-(4-methylphenoxy)octansäure
8-(4-Chlorphenoxy)-2-(4-methylphenylthio)octansäure

**Beispiel 1**

8-Methoxy-2-(4-methylphenylsulfonyl)-octansäure

Man versetzt eine Lösung von 1,38 g (60 mmol) Natrium in 500 ml abs. Ethanol unter Rühren bei Siedehitze mit 14,5 g (60 mmol) 4-Methylphenylsulfonylessigsäureethylester. Dann rührt man zur Vervollständigung der Salzbildung 1 Stunde unter Rückfluß nach und tropft anschließend eine Lösung von 11,7 g (60 mmol) 6-Methoxyhexylbromid in 100 ml abs. Ethanol zu.

Man rührt noch 5 Stunden bei Rückflußtemperatur nach, dampft das Lösungsmittel ab und versetzt den Rückstand mit Wasser. Die organischen Bestandteile werden mit Ether extrahiert und die Extrakte getrocknet und eingedampft. Den Rückstand chromatographiert man mit einem Gemisch von Heptan und Butanon-2 (2 : 1) an Kieselgel. Man erhält 12,8 g (64% d. Th.) 8-Methoxy-2-(4-methylphenylsulfonyl)octansäureethylester, farbloses Öl.

2,5 g (7 mmol) dieses Esters werden in Gemisch von 15 ml 1 N Kalilauge und 50 ml Methanol 3 Stunden bei 40°C gerührt. Dann wird das Methanol abdestilliert, der Rückstand mit Wasser verdünnt und mit Ether die Neutralanteile ausgewaschen. Die wässrige Lösung wird anschließend mit Kohle geklärt, angesäuert und das abgeschiedene Produkt mit Ether extrahiert. Die Etherextrakte werden getrocknet und eingedampft. Das zurückbleibende Öl wird mit einem Gemisch von 0,58 g (7 mmol) Natriumhydrogencarbonat, 30 ml Ethanol und 30 ml Wasser 1 Stunde gerührt und die erhaltene klare Lösung im Vakuum eingedampft. Der Rückstand wird unter Heptan verrieben. Man erhält 1,8 g (77% d. Th.) Natrium-[8-Methoxy-2-(4-methylphenylsulfonyl)]octanoat, Fp. 135-139°C.

**Beispiel 2**

8-Methylthio-2-(4-methylphenylsulfonyl)-octansäureethylester

In analoger Weise wie in Beispiel 1 beschrieben erhält man aus 21,8 g (90 mmol) 4-Methylphenylsulfonylessigsäureethylester, 19,0 g (90 mmol) 6-Methylthiohexylbromid und 2,07 g (90 mmol) Natrium 22,5 g (67% d. Th.) 8-Methylthio-2-(4-methylphenylsulfonyl)-octansäureethylester, farbloses Öl.

**Beispiel 3**

8-Methylsulfonyl-2-(4-methylphenylsulfonyl)-octansäure-ethylester

Man rührt ein Gemisch von 4,0 g (10,7 mol) 8-Methylthio-2-(4-methylphenylsulfonyl)-octansäureethylester, 5,35 ml einer 4-molaren Lösung von Trifluorperessigsäure in Trifluoressigsäure und 7,5 ml Trifluoressigsäure 4 h bei 0°C.

Dann wird das Lösungsmittel im Vakuum abdestilliert und der Rückstand in Ether aufgenommen. Man wäscht diese Lösung mit wässrigem Natriumhydrogencarbonat, trocknet und dampft ein. Der Rückstand wird mit einem Gemisch von Toluol und Dioxan (5 : 1) an Kieselgel chromatographiert. Man erhält 3,2 g (60% d. Th.) 8-Methylsulfonyl-2-(4-methylphenylsulfonyl)-octansäureethylester, farbloses Öl.

**Beispiel 4**

8-(4-Chlorphenyl)-2-(3-methylphenylsulfonyl)ocantsäure

Man erhitzt ein Gemisch von 87,5 g (0,49 mol) Natrium-(3-methylphenyl)sulfinat, 90,2 g (0.54 mol) Bromessigsäureethylester und 400 ml Ethanol.

Abs. 5 Stunden auf Rückflußtemperatur. Dann destilliert man das Lösungsmittel im Vakuum ab und nimmt den Rückstand in Wasser auf. Die organischen Bestandteile werden mit Ether extrahiert und nach üblicher Aufarbeitung im Hochvakuum destilliert. Man erhält 102,1 g (86% d. Th.) 3-Methylphenylsulfonylessigsäureethylester, Sdp. 157 bis 164°C/0,5 mbar.

Zu einer Lösung von 36 mmol Natriummethylat in 150 ml abs. Ethanol wird unter Rühren bei Rückflußtemperatur eine Lösung von 8,8 g (36 mmol) 3-Methylphenylsulfonylessigsäurethylester in 50 ml abs. Ethanol getropft und das Gemisch 1 Stunde nachgerührt. Dann gibt man 10,0 g (36 mmol) 6-(4-Chlorphenyl)hexylbromid zu und erhitzt erneut 10 Stunden auf Rückflußtemperatur. Anschließend arbeitet man wie in Beispiel 1 beschrieben auf und chromatographiert den Rückstand mit einem Gemisch von Heptan und Butanon –2 (2 : 1) an Kieselgel. Man erhält 11,3 g (72% d. Th.) 8-(4-Chlorphenyl)-2-1(3-methylphenylsulfonyl)octansäureethylester, farbloses Öl.

4,8 g (11 mmol) dieses Esters werden in einem Gemisch von 25 ml 1 N Kalilauge und 300 ml Methanol, wie in Beispiel 1 beschrieben, verseift. Man erhält 4,3 g (95% d. Th.) 8-(4-Chlorphenyl)-2-(3-methylphenylsulfonyl)octansäure, farbloses Öl.

**Beispiel 5**

4-[2-(4-Chlorophenyl)ethoxy]-2-(4-methylphenylsulfonyl)butansäure

In analoger Weise wie in Beispiel 1 beschrieben erhält man aus 13,2 g (50 mmol) 2-[2-(4-Chlorphenyl)-ethoxy]ethylbromid (Sdp. 170°C/20 mbar), 12,1 g (50 mmol) 4-Methylphenylsulfonylessigsäureethylester und 1,15 g (50 mmol) Natrium 19,0 g (89% d. th.) 4-[2-(4-Chlorphenyl)ethoxy]-2-(4-methylphenylsulfonyl)-butansäureethylester, farbloses Öl und daraus 9,24 g (75% d. Th.) Natrium-4-[2-(4-Chlorphenyl)ethoxy]-2-(4-methylphenylsulfonyl)]butanoat, Fp. 70 bis 72°C (amorph.).

## Ansprüche

**Patentansprüche für die Vertragsstaaten : AT, BE, CH, DE, FR, GB, IT, LI, LU, NL, SE**

1. Carbonsäurederivate der allgemeinen Formel I

```
R1-A-CH-COOH          (I),
    |
    Y-B-R2
```

in welcher

$R_1$ einen $C_1$-$C_8$-Alkoxy-, $C_1$-$C_8$-Alkylthio-, $C_1$-$C_8$-Alkylsulfinyl-, $C_1$-$C_8$-Alkylsulfonyl- oder $C_1$-$C_8$-Alkylamino-

rest, einen $C_3$-$C_{10}$-Cycloalkyl- oder $C_3$-$C_{10}$-Cycloalkoxyrest, einen Phenyl-, Phenyloxy-, Phenylthio-, Phenylsulfinyl-, Phenylsulfonyl- oder Phenylaminogruppe, wobei die Phenylteile der zuvor genannten Reste ein- oder mehrfach durch Hydroxy-, Halogen-, $C_1$-$C_6$-Alkyl-, $C_1$-$C_6$-Alkoxy-, Trifluormethyl-, Cyano-, Nitro-, Amino-, $C_1$-$C_6$-Alkylamino- oder Di-$C_1$-$C_6$-alkylaminogruppen substituiert sein können,

$R_2$ einen Phenylrest, der ein- oder mehrfach durch Halogen-, $C_1$-$C_6$-Alkyl-, $C_1$-$C_6$-Alkoxy und Trifluormethylgruppen substituiert sein kann, oder für den Fall, daß B eine Alkylengruppe ist, auch Wasserstoff,

A die Gruppen $-(CH_2)_o-$ mit o = 3-10 oder $-(CH_2)_p$-X-$(CH_2)_q$ mit p = 2-8 und q = 1-6, wobei o, p, q ganze Zahlen bedeuten und die Summe von p und q nicht größer als 10 sein darf und X ein Sauerstoff- oder Schwefelatom oder die Gruppe –NH– bedeutet, oder, falls $R_1$ einen wie un der Definition von $R_1$ angegebenen Phenylrest darstellt, A auch die Gruppen $-CH_2$-X-$(CH_2)_q$, $-CH{\equiv}CH-CH_2-$, $-C{\equiv}C-CH_2-$, $-C{\equiv}C-(CH_2)_p-$, $-CH{=}CH-CH_2$-X-$(CH_2)_q$, $-C{\equiv}C-CH_2$-X-$(CH_2)_q$, wobei p, q und X die oben angegebene Definition besitzen,

Y die Gruppe $-S(O)_n-$ oder –O– mit n = 0, 1 oder 2 und

B einen Valenzstrich oder die Gruppe $-(CH_2)_r-$ mit r = 1-18, $-CH_2-CH{=}CH-$, $-CH_2-C{\equiv}C-$ oder $-CH{=}CH-$ bedeuten mit Ausnahme der Verbindungen 2-Phenoxy-5-phenylvaleriansäure, 2-Phenylthio-5-phenyl-4-pentensäure und 2-Phenylsulfonyl-5-phenyl-4-pentensäure,

sowie deren physiologisch unbedenklichen Salze, Ester und Amide.

2. Carbonsäurederivate der Formel I gemäß Anspruch 1, in der A die Gruppe $-(CH_2)_o-$ mit o = 4, 5 oder 6 bedeutet.

3. Carbonsäurederivate der Formel I gemäß Anspruch 1, in der

$R_1$ einen Phenyl- oder Phenoxyrest bedeutet, der gegebenenfalls ein- oder mehrfach durch Halogen-, $C_1$-$C_6$-Alkyl-, $C_1$-$C_6$-Alkoxy- und Trifluormethylgruppen substituiert sein kann.

4. Carbonsäurederivate der Formel I gemäß Anspruch 1, in der

$R_2$ einen Phenyl-, 2-Methylphenyl-, 3-Methylphenyl-, 4-Methylphenyl, 4-tert.-Butylphenyl-, 4-Methoxy- und 3-Trifluormethylphenylrest bedeutet.

5. Carbonsäurederivate gemäß Anspruch 1 ausgewählt aus der Gruppe der folgenden Verbindungen :

8-Methoxy-2-(4-methylphenylsulfonyl)-octansäure
8-Methylthio-2-(4-methylphenylsulfonyl)-octansäure
8-Methylsulfonyl-2-(4-methylphenylsulfonyl)-octansäure
8-(4-Chlorphenyl)-2-(3-methylphenylsulfonyl)-octansäure
4-[2-(4-Chlorophenyl)ethoxy]-2-(4-methylphenylsulfonyl)-butansäure
7-Phenyl-2-[2-(4-t-butylphenyl)ethylsulfonyl]heptansäure
8-Phenyl-2-(4-t-butylphenoxy)octansäure
8-Phenyl-2-(4-t-butylphenylthio)octansäure
8-Phenyl-2-(4-ethylphenylsulfonyl)octansäure.
8-Phenyl-2-(4-propylphenylsulfonyl)octansäure
8-Phenyl-2-(4-t-butylphenylsulfonyl)octansäure
7-(4-Chlorphenyl)-2-(4-t-butylphenoxy)heptansäure
7-(4-Chlorphenyl)-2-(4-t-butylphenylthio)heptansäure
7-(4-Chlorphenyl)-2-(4-t-butylphenylsulfonyl)heptansäure
8-(4-Chlorphenyl)-2-(4-t-butylphenoxy)octansäure
8-(4-Chlorphenyl)-2-(4-t-butylphenylthio)octansäure
8-(4-Chlorphenyl)-2-(4-ethylphenylsulfonyl)octansäure
8-(4-Chlorphenyl)-2-(4-propylphenylsulfonyl)octansäure
8-(4-Chlorphenyl)-2-(4-t-butylphenylsulfonyl)octansäure
7-(4-Methoxyphenyl)-2-(4-t-butylphenoxy)heptansäure
7-(4-Methoxyphenyl)-2-(4-t-butylphenylthio)heptansäure
7-(4-Methoxyphenyl)-2-(4-t-butylphenylsulfonyl)heptansäure
6-Phenoxy-2-(4-t-butylphenoxy)hexansäure
6-(4-Chlorphenoxy)-2-(4-methylphenoxy)hexansäure
6-(4-Chlorphenoxy)-2-(4-t-butylphenoxy)hexansäure

6. Arzneimittel, enthaltend mindestens eine Verbindung gemäß Anspruch 1-5 oder die Verbindung 2-Phenylthio-S-phenyl-4-pentensäure oder 2-Phenylsulfonyl-5-phenyl-4-pentensäure bzw. deren physiologisch unbedenkliche Salze, Ester oder Amide, neben üblichen Träger- und Hilfsstoffen.

7. Arzeimittel gemäß Anspruch 6 mit antidiabetischer Wirkung.

8. Verwendung von Verbindungen gemäß Anspruch 1-5 zur Herstellung von Arzneimitteln mit antidiabetischer Wirkung.

**Patentansprüche für die Vertragsstaaten : ES, GR**

1. Verfahren zur Herstellung von Carbonsäurederivaten der allgemeinen Formel I

$$\begin{array}{l} R_1-A-CH-COOH \\ \qquad\quad | \\ \qquad\quad Y-B-R_2 \end{array} \qquad (I),$$

in welcher

$R_1$ einen $C_1$-$C_8$-Alkoxy-, $C_1$-$C_8$-Alkylthio-, $C_1$-$C_8$-Alkylsulfinyl-, $C_1$-$C_8$-Alkylsulfonyl- oder $C_1$-$C_8$-Alkylaminorest, einen $C_3$-$C_{10}$-Cycloalkyl- oder $C_3$-$C_{10}$-Cycloalkoxyrest, einen Phenyl-, Phenyloxy-, Phenylthio-, Phenylsulfinyl-, Phenylsulfonyl- oder Phenylaminogruppe, wobei die Phenylteile der zuvor genannten Reste ein- oder mehrfach durch Hydroxy-, Halogen-, $C_1$-$C_6$-Alkyl-, $C_1$-$C_6$-Alkoxy-, Trifluormethyl-, Cyano-, Nitro-, Amino-, $C_1$-$C_6$-Alkylamino- oder Di-$C_1$-$C_6$-alkylaminogruppen substituiert sein können,

$R_2$ einen Phenylrest, der ein- oder mehrfach durch Halogen-, $C_1$-$C_6$-Alkyl-, $C_1$-$C_6$-Alkoxy und Trifluormethylgruppen substituiert sein kann, oder für den Fall, daß B eine Alkylengruppe ist, auch Wasserstoff,

A die Gruppen $-(CH_2)_o-$ mit o = 3-10 oder $-(CH_2)_p$-X-$(CH_2)_q$ mit p = 2-8 und q = 1-6, wobei o, p, q ganze Zahlen bedeuten und die Summe von p und q nicht größer als 10 sein darf und X ein Sauerstoff- oder Schwefelatom oder die Gruppe –NH– bedeutet, oder, falls $R_1$ einen wie in der Definition von $R_1$ angegebenen Phenylrest darstellt, A auch die Gruppen $-CH_2$-X-$(CH_2)_q$, $-CH=CH-CH_2-$, $-C\equiv C-CH_2-$, $-C\equiv C-(CH_2)_p-$, $-CH=CH-CH_2$-X- $(CH_2)$q, $-C\equiv C-CH_2$-X-$(CH_2)_q$, wobei p, q und X die oben angegebene Definition besitzen,

Y die Gruppe $-S(O)_n-$ oder –O– mit n = 0, 1 oder 2 und

B einen Valenzstrich oder die Gruppe $-(CH_2)_r-$ mit r = 1-18, $-CH_2-CH=CH-$, $-CH_2-C\equiv C-$ oder –CH=CH– bedeuten mit Ausnahme der Verbindungen 2-Phenoxy-5-phenylvaleriansäure, 2-Phenylthio-5-phenyl-4-pentensäure und 2-Phenylsulfonyl-5-phenyl-4-pentensäure,

sowie deren physiologisch unbedenklichen Salze, Ester und Amide, dadurch gekennzeichnet, daß man

A) eine Gruppe der allgemeinen Formel II

$$\begin{array}{l} Z-CH-W \\ \quad\;\; | \\ \quad\;\; X \end{array} \qquad (II),$$

in welcher

W $-COOR_3$ oder eine andere in die Carboxylfunktion überführbare Gruppe

X ein reaktiver Rest und

Z Wasserstoff oder die Gruppe $R_1$-A-, in welcher $R_1$ und A die oben angegebenen Beteutungen besitzen und

$R_3$ niederes Alkyl

bedeutet,

mit einer Gruppe der allgemeinen Formel III

$$R_2\text{-B-YH}, \quad (III)$$

in welcher $R_2$, B und Y die oben angegebenen Bedeutungen besitzen, zu einer Verbindung der allgemeinen Formel IV

$$\begin{array}{l} Z-CH-W \\ \quad\;\; | \\ \quad\;\; Y-B-R_2 \end{array} \qquad (IV)$$

umsetzt und im Anschluß daran gegebenenfalls

a) die Gruppe W in die freie Carboxylfunktion, einen physiologisch verträglichen Ester oder ein Amid umwandelt,

b) für den Fall, daß Y ein Schwefelatom darstellt, die Gruppe Y in die Gruppe SO oder $SO_2$ umwandelt oder

c) für den Fall, daß Z Wasserstoff bedeutet, entweder

C1) mit einer Verbindung der allgemeinen Formel V

$$R_1\text{-A-X} \quad (V)$$

in welcher $R_1$ und A die oben angegebenen Bedeutungen besitzen und X eine reaktive Gruppe bedeutet, alkyliert oder

C2) mit einer Verbindung der allgemeinen Formel VI

$$R_1\text{-A'-CHO} \quad (V')$$

in welcher A' einen um die $CH_2$-Gruppe verkürzten Alkylenrest A bedeutet und

$R_1$ die oben angegebene Bedeutung besitzt, kondensiert und im Anschluß an die Kondensation die entstandene Doppelbindung hydriert und die aus den vorgenannten Verfahrensschritten C1) oder C2) erhaltenen Verbindungen IV mit Z = $R_1$-A– in der oben angegebenen Weise an den Gruppen W oder Y umwandelt oder

B) eine Verbindung der allgemeinen Formel VI

```
R1-A-CH-W                                (VI),
     |
     YH
```

in welcher $R_1$, A, Y und W die oben angegebenen Bedeutungen besitzen, mit einer Gruppe der allgemeinen Formel VII

$$R_2\text{-B-X} \quad (VII)$$

in welcher $R_2$, B und X die oben angegebenen Bedeutungen besitzen, zu einer Verbindung der Formel IV umsetzt und diese gegebenenfalls in der oben angegebenen Weise an den Gruppen W und Y umwandelt und anschließend die erhaltenen Verbindungen gewünschtenfalls in physiologisch unbedenkliche Amide, Ester oder Salze überführt.

2. Verfahren zur Herstellung von Carbonsäurederivaten der Formel I gemäß Anspruch 1, in der A die Gruppe $-(CH_2)_o-$ mit o = 4, 5 oder 6 bedeutet.

3. Verfahren zur Herstellung von Carbonsäurederivaten der Formel I gemäß Anspruch 1, in der $R_1$ einen Phenyl- oder Phenoxyrest bedeutet, der gegebenenfalls ein- oder mehrfach durch Halogen-, $C_1$-$C_6$-Alkyl-, $C_1$-$C_6$-Alkoxy- und Trifluormethylgruppen substituiert sein kann.

4. Verfahren zur Herstellung von Carbonsäurederivaten der Formel I gemäß Anspruch 1, in der $R_2$ einen Phenyl-, 2-Methylphenyl-, 3-Methylphenyl-, 4-Methylphenyl, 4-tert.-Butylphenyl-, 4-Methoxy- und 3-Trifluormethylphenylrest bedeutet.

5. Verfahren zur Herstellung von Carbonsäurederivaten gemäß Anspruch 1 ausgewählt aus der Gruppe der folgenden Verbindungen :

8-Methoxy-2-(4-methylphenylsulfonyl)-octansäure
8-Methylthio-2-(4-methylphenylsulfonyl)-octansäure
8-Methylsulfonyl-2-(4-methylphenylsulfonyl)-octansäure
8-(4-Chlorphenyl)-2-(3-methylphenylsulfonyl)-octansäure
4-[2-(4-Chlorophenyl)ethoxy]-2-(4-methylphenylsulfonyl)-butansäure
7-Phenyl-2-[2-(4-t-butylphenyl)ethylsulfonyl]heptansäure
8-Phenyl-2-(4-t-butylphenoxy)octansäure
8-Phenyl-2-(4-t-butylphenylthio)octansäure
8-Phenyl-2-(4-ethylphenylsulfonyl)octansäure.
8-Phenyl-2-(4-propylphenylsulfonyl)octansäure
8-Phenyl-2-(4-t-butylphenylsulfonyl)octansäure

7-(4-Chlorphenyl)-2-(4-t-butylphenoxy)heptansäure
7-(4-Chlorphenyl)-2-(4-t-butylphenylthio)heptansäure
7-(4-Chlorphenyl)-2-(4-t-butylphenylsulfonyl)heptansäure
8-(4-Chlorphenyl)-2-(4-t-butylphenoxy)octansäure
8-(4-Chlorphenyl)-2-(4-t-butylphenylthio)octansäure
8-(4-Chlorphenyl)-2-(4-ethylphenylsulfonyl)octansäure
8-(4-Chlorphenyl)-2-(4-propylphenylsulfonyl)octansäure
8-(4-Chlorphenyl)-2-(4-t-butylphenylsulfonyl)octansäure
7-(4-Methoxyphenyl)-2-(4-t-butylphenoxy)heptansäure
7-(4-Methoxyphenyl)-2-(4-t-butylphenylthio)heptansäure
7-(4-Methoxyphenyl)-2-(4-t-butylphenylsulfonyl)heptansäure
6-Phenoxy-2-(4-t-butylphenoxy)hexansäure
6-(4-Chlorphenoxy)-2-(4-methylphenoxy)hexansäure
6-(4-Chlorphenoxy)-2-(4-t-butylphenoxy)hexansäure

6. Verfahren zur Herstellung von Arzneimitteln enthaltend als Wirkstoff mindestens eine Verbindung der Formel I gemäß einen der Ansprüche 1-5 oder die Verbindung 2-Phenylthio-5-phenyl-4-pentensäure oder 2-phenylsulfonyl-5-phenyl-4-pentensäure bzw. deren physiologisch unbedenklichen Salze, Ester oder Amide, dadurch gekennzeichnet, daß man den Wirkstoff zusammen mit üblichen Träger- und Hilfsstoffen zu Auszueimitteln verarbeitet.

**Revendications**

**Revendications pour les Etats Contractants : AT, BE, CH, DE, FR, GB, IT, LI, LU, NL, SE**

1. Dérivés d'acides carboxyliques de formule générale I

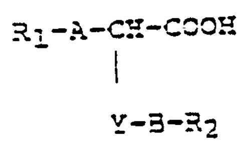

(I) ,

dans lesquels

$R_1$ représente un groupe alcoxy en $C_1$-$C_8$, alkylthio en $C_1$-$C_8$, alkylsulfinyle en $C_1$-$C_8$, alkylsulfonyle en $C_1$-$C_8$ ou alkylamino en $C_1$-$C_8$, un groupe cycloalkyle en $C_3$-$C_{10}$ ou cycloalcoxy en $C_3$-$C_{10}$, un groupe phényle, phényloxy, phénylthio, phénylsulfinyle, phénylsulfonyle ou phénylamino, la partie phényle des groupes mentionnés ci-dessus pouvant être substituée une ou plusieurs fois par un groupe hydroxy, un atome d'halogène, un groupe alkyle en $C_1$-$C_6$, alcoxy en $C_1$-$C_6$, trifluorométhyle, cyano, nitro, amino, alkyl($C_1$-$C_8$)amino ou dialkyl($C_1$-$C_6$)amino,

$R_2$ peut représenter un groupe phényle, qui peut être substitué une ou plusieurs fois par un groupe halogène, alkyle en $C_1$-$C_6$, alcoxy en $C_1$-$C_6$, et trifluorométhyle, ou dans le cas où B représente un groupe alkylène, $R_2$ peut également représenter l'hydrogène,

A représente les groupes $-(CH_2)q-$ , avec o = 3-10 ou $-(CH_2)_p$-X-$(CH_2)_q$, avec p = 2-8 et q = 1-6, o, p et q représentant des nombres entiers, et la somme de p et de q ne devant pas dépasser 10, et X représente un atome d'oxygène ou de soufre ou le groupe -NH- , ou dans le cas où $R_1$ représente un groupe phényle tel qu'indiqué dans la définition de $R_1$, A peut également représenter les groupes $-(CH_2)$-X-$(CH_2)_q-$ , -CH=CH-$CH_2$- , -C≡C-$CH_2$- , -C≡C-$(CH_2)_p$, -CH=CH-$CH_2$-X-$(CH_2)_q$, -C≡C-$CH_2$-X-$(CH_2)_q$, où p, q et X ont les significations mentionnées ci-dessus,

Y représente le groupe $-S(O)_n-$ ou -O- , avec n = 0, 1 ou 2 et

B représente une valence libre ou le groupe $-(CH2)_r-$ , avec r = 1-18, $-CH_2$=CH=CH- , $-CH_2$-C≡C- ou -CH=CH- ,

à l'exception des composés acide 2-phénoxy-5-phénylvalérique, acide 2-phénylthio-5-phényl-4-penténoïque et acide 2-phénylsulfonyl-5-phényl-4-penténoïque,

ainsi que leurs sels, esters et amides physiologiquement acceptables.

2. Dérivés d'acides carboxyliques de formule I selon la revendication 1, où A représente le groupe $-(CH_2)_o-$ , avec o = 4, 5 ou 6.

3. Dérivés d'acides carboxyliques de formule I selon la revendication 1, où

$R_1$ représente un groupe phényle ou phénoxy, qui peut être éventuellement substitué une ou plusieurs fois par un atome d'halogène, un groupe alkyle en $C_1$-$C_6$, alcoxy en $C_1$-$C_6$, et trifluorométhyle.

4. Dérivés d'acides carboxyliques de formule I selon la revendication 1, où

$R_2$ représente un groupe phényle, 2-méthylphényle, 3 -méthylphényle, 4-méthylphényle, 4-tert.-butylphényle, 4-méthoxy- et 3-trifluorométhylphényle.

5. Dérivés d'acides carboxyliques selon la revendication 1, choisis dans le groupe constitué par les composés suivants :

Acide 8-méthoxy-2-(4-méthylphénylsulfonyl)-octanoïque,
Acide 8-méthylthio-2-(4-méthylphénylsulfonyl)-octanoïque,
Acide 8-méthylsulfonyl-2-(4-méthylphénylsulfonyl) -octanoïque,
Acide 8-(4-chlorophényl)-2-(3-méthylphénylsulfonyl)-octanoïque,
Acide 4-[2-(4-chlorophényl)éthoxy]-2-(4-méthylphénylsulfonyl)-butanoïque,
Acide 7-phényl-2-[2-(4-t-butylphényl)éthylsulfonyl]heptanoïque,
Acide 8-phényl-2-(4-t-butylphénoxy)octanoïque,
Acide 8-phényl-2-(4-t-butylphénylthio)octanoïque,
Acide 8-phényl-2-(4-éthylphénylsulfonyl)octanoïque,
Acide 8-phényl-2-(4-propylylphénylsulfonyl)octanoïque,
Acide 8-phényl-2-(4-t-butylphénylsulfonyl)octanoïque,
Acide 7-(4-chlorophényl)-2-(4-t-butylphénoxy)heptanoïque,
Acide 7-(4-chlorophényl)-2-(4-t-butylphénylthio)heptanoïque,
Acide 7-(4-chlorophényl)-2-(4-t-butylsulfonyl)heptanoïque,
Acide 8-(4-chlorophényl)-2-(4-t-butylphénoxy)octanoïque,
Acide 8-(4-chlorophényl)-2-(4-t-butylphénylthio)octanoïque,
Acide 8-(4-chlorophényl)-2-(4-éthylphénylsulfonyl)octanoïque,
Acide 8-(4-chlorophényl)-2-(4-propylphénylsulfonyl)octanoïque,
Acide 8-(4-chlorophényl)-2-(4-t-butylphénylsulfonyl)octanoïque,
Acide 7-(4-méthoxyphényl)-2-(4-t-butylphénoxy)heptanoïque,
Acide 7-(4-méthoxyphényl)-2-(4-t-butylphénylthio)heptanoïque,
Acide 7-(4-méthoxyphényl)-2-(4-t-butylphénylsulfonyl)heptanoïque,
Acide 6-phénoxy-2-(4-t-butylphénoxy)hexanoïque,
Acide 6-(4-chlorophénoxy)-2-(4-méthylphénoxy)hexanoïque,
Acide 6-(4-chlorophénoxy)-2-(4-butylphénoxy)hexanoïque.

6. Médicament contenant au moins un composé selon les revendications 1 à 5, ou le composé acide 2-phénylthio-5-phényl-4-penténoïque ou le composé acide 2-phénylsulfonyl-5-phényl-4-penténoïque, ou leurs sels, esters ou amides physiologiquement acceptables, en plus des véhicules et adjuvants usuels.

7. Médicament selon la revendication 6, ayant une activité antidiabétique.

8. Utilisation des composés selon les revendications 1 à 5, pour la préparation de médicaments ayant une activité antidiabétique.

**Revendications pour les Etats Contractants : ES, GR**

1. Procédé pour la préparation de dérivés d'acides carboxyliques de formule générale I

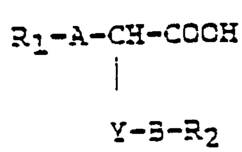

(I) ,

dans lesquels

$R_1$ représente un groupe alcoxy en $C_1$-$C_8$, alkylthio en $C_1$-$C_8$, alkylsulfinyle en $C_1$-$C_8$, alkylsulfonyle en $C_1$-$C_8$ ou alkylamino en $C_1$-$C_8$, un groupe cycloalkyle en $C_3$-$C_{10}$ ou cycloalcoxy en $C_3$-$C_{10}$, un groupe phényle, phényloxy, phénylthio, phénylsulfinyle, phénylsulfonyle ou phénylamino, la partie phényle des groupes mentionnés ci-dessus pouvant être substitués une ou plusieurs fois par un groupe hydroxy, un atome d'halogène, un groupe alkyle en $C_1$-$C_6$, alcoxy en $C_1$-$C_6$, trifluorométhyle, cyano, nitro, amino, alkyl($C_1$-$C_6$)amino ou dialkyl($C_1$-$C_6$)amino,

$R_2$ peut représenter un groupe phényle, qui peut être substitué une ou plusieurs fois par un groupe halogène, alkyle en $C_1$-$C_6$, alcoxy en $C_1$-$C_6$, et trifluorométhyle, ou dans le cas où B représente un groupe alkylène,

$R_2$ peut également représenter l'hydrogène,

A représente les groupes $-(CH_2)_o-$, avec o = 3-10 ou $-(CH_2)_p$-X-$(CH_2)_q-$, avec p = 2-8 et q = 1-6, o, p et q représentant des nombres entiers, et la somme de p et de q ne devant pas dépasser 10, et X représente un atome d'oxygène ou de soufre ou le groupe –NH–, ou dans le cas où $R_1$ représente un groupe phényle tel qu'indiqué dans la définition de $R_1$, A peut également représenter les groupes $-(CH_2)$-X-$(CH_2)_q-$, –CH=CH-$CH_2$–, –C≡C-$CH_2$–, –C≡C-$(CH_2)_p$, –CH≡CH-$CH_2$-X-$(CH_2)_q$, –C=C-$CH_2$-X-$(CH_2)_q$, où p, q et X ont les significations mentionnées ci-dessus,

Y représente le groupe –S(O)n– ou –O–, avec n = 0, 1 ou 2 et

B représente une valence libre où le groupe $-(CH_2)_r-$, avec r = 1-18, $-CH_2$=CH=CH–, $-CH_2$-C≡C– ou –CH=CH–,

à l'exception des composés acide 2-phénoxy-5-phénylvalérique, acide 2-phénylthio-5-phényl-4-penténoïque et acide 2-phénylsulfonyl-5-phényl-4-penténoïque,

ainsi que leurs sels, esters et amides physiologiquement acceptables,

caractérisé en ce que l'on fait réagir

A) un groupe de formule générale II

```
Z-CH-W          (II),
  |
  X
```

dans laquelle

W représente le groupe $-COOR_3$ ou un autre groupe pouvant être transformé en fonction carboxyle,

X représente un groupe réactif et

Z représente l'hydrogène ou le groupe $R_1$-A–, dans lequel $R_1$ et A ont les significations mentionnées ci-dessus, et

$R_3$ représente un groupe alkyle inférieur, avec un groupe de formule générale III

$$R_2\text{-B-YH} \quad (III)$$

dans laquelle $R_2$, B et Y ont les significations mentionnées ci-dessus, en un composé de formule générale IV

```
Z-CH-W
  |
  Y-B-R2        (IV)
```

et ensuite éventuellement,

a) on transforme le groupe W en la fonction carboxyle libre, un ester ou un amide physiologiquement acceptables,

b) dans le cas où Y représente un atome de soufre, on transforme le groupe Y en le groupe SO ou $SO_2$, ou

c) dans le cas où Z représente l'hydrogène, soit

C1) on effectue une alkylation avec un composé de formule générale V

$$R_1\text{-A-X} \quad (V)$$

dans laquelle $R_1$ et A ont les significations mentionnées ci-dessus et X représente un groupe réactif, soit C2) on effectue une condensation avec un composé de formule générale VI

$$R_1\text{-A'-CHO,} \quad (V')$$

dans laquelle A' représente un groupe alkylène A raccourci d'un groupe $CH_2$, et

$R_1$ a la signification mentionnée plus haut, et après la condensation, on hydrogène la double liaison formée et on transforme les composés IV, obtenus dans les étapes de procédé C1) ou C2) mentionnées ci-dessus,

dans lesquels Z = $R_1A-$ , selon le mode opératoire indiqué ci-dessus, en les groupes W ou Y, ou

B) on fait réagir un composé de formule générale VI

$$\begin{array}{l} R_1-A-CH-W \\ \qquad\quad | \\ \qquad\quad YH \end{array} \qquad (VI)$$

dans laquelle $R_1$, A, Y et W ont les significations mentionnées ci-dessus, avec un groupe de formule générale VII

$$R_2\text{-}B\text{-}X \quad (VII)$$

dans laquelle $R_2$, B et X ont les significations mentionnées ci-dessus, en un composé de formule IV et l'on transforme éventuellement celui-ci, de la manière indiquée ci-dessus, en les groupes W et Y et ensuite, on transforme éventuellement les composés obtenus en leurs amides, esters ou sels physiologiquement acceptables.

2. Procédé pour la préparation des dérivés d'acides carboxyliques de formule I selon la revendication 1, où A représente le groupe $-(CH_2)_o-$ , avec o = 4, 5 ou 6.

3. Procédé pour la préparation des dérivés d'acides carboxyliques de formule I selon la revendication 1, où

$R_1$ représente un groupe phényle ou phénoxy, qui peut être éventuellement substitué une ou plusieurs fois par un atome d'halogène, un groupe alkyle en $C_1$-$C_6$, alcoxy en $C_1$-$C_6$, et trifluorométhyle.

4. Procédé pour la préparation des dérivés d'acides carboxyliques de formule I selon la revendication 1, où

$R_2$ représente un groupe phényle, 2-méthylphényle, 3-méthylphényle, 4-méthylphényle, 4-tert.-butylphényle, 4-méthoxy- et 3-trifluorométhylphényle.

5. Procédé pour la préparation des dérivés d'acides carboxyliques selon la revendication 1, choisis dans le groupe constitué par les composés suivants :

Acide 8-méthoxy-2-(4-méthylphénylsulfonyl)-octanoïque,
Acide 8-méthylthio-2-(4-méthylphénylsulfonyl)-octanoïque,
Acide 8-méthylsulfonyl-2-(4-méthylphénylsulfonyl)-octanoïque,
Acide 8-(4-chlorophényl)-2-(3-méthylphénylsulfonyl)-octanoïque,
Acide 4-[2-(4-chlorophényl)éthoxy]-2-(4-méthylphénylsulfonyl)butanoïque,
Acide 7-phényl-2-[2-(4-t-butylphényl)éthylsulfonyl]heptanoïque,
Acide 8-phényl-2-(4-t-butylphénoxy)octanoïque,
Acide 8-phényl-2-(4-t-butylphénylthio)octanoïque,
Acide 8-phényl-2-(4-éthylphénylsulfonyl)octanoïque,
Acide 8-phényl-2-(4-propylylphénylsulfonyl)octanoïque,
Acide 8-phényl-2-(4-t-butylphénylsulfonyl)octanoïque,
Acide 7-(4-chlorophényl)-2-(4-t-butylphénoxy)heptanoïque,
Acide 7-(4-chlorophényl)-2-(4-t-butylphénylthio)heptanoïque,
Acide 7-(4-chlorophényl)-2-(4-t-butylsulfonyl)heptanoïque,
Acide 8-(4-chlorophényl)-2-(4-t-butylphénoxy)octanoïque,
Acide 8-(4-chlorophényl)-2-(4-t-butylphénylthio)octanoïque,
Acide 8-(4-chlorophényl)-2-(4-éthylphénylsulfonyl)octanoïque,
Acide 8-(4-chlorophényl)-2-(4-propylphénylsulfonyl)octanoïque,
Acide 8-(4-chlorophényl)-2-(4-t-butylphénylsulfonyl)octanoïque,
Acide 7-(4-méthoxyphényl)-2-(4-t-butylphénoxy)heptanoïque,
Acide 7-(4-méthoxyphényl)-2-(4-t-butylphénylthio)heptanoïque,
Acide 7-(4-méthoxyphényl)-2-(4-t-butylphénylsulfonyl)heptanoïque,
Acide 6-phénoxy-2-(4-t-butylphénoxy)hexanoïque,
Acide 6-(4-chlorophénoxy)-2-(4-méthylphénoxy)hexanoïque,
Acide 6-(4-chlorophénoxy)-2-(4-butylphénoxy)hexanoïque.

6. Procédé pour la préparation d'un médicament contenant, comme composant actif, au moins un composé selon les revendications 1 à 5, ou le composé acide 2-phénylthio-5-phényl-4-penténoïque ou le composé acide 2-phénylsulfonyl-5-phényl-4-penténoïque, ou leurs sels, esters ou amides physiologiquement acceptables,

caractérisé en ce qu'à l'aide de véhicules et adjuvants usuels, on transforme le composant actif en un médicament.

## Claims

**Claims for the Contracting States : AT, BE, CH, DE, FR, GB, IT, LI, LU, NL, SE**

1. Carboxylic acid derivatives of the general formula I

$$\begin{array}{l} R_1\text{-}A\text{-}CH\text{-}COOH \\ \qquad\quad | \\ \qquad\quad Y\text{-}B\text{-}R_2 \end{array} \qquad (I)$$

in which $R_1$ signifies a $C_1$-$C_8$-alkoxy, $C_1$-$C_8$-alkylthio, $C_1$-$C_8$-alkylsulphinyl, $C_1$-$C_8$-alkylsulphonyl or $C_1$-$C_8$-alkylamino radical, a $C_3$-$C_{10}$-cycloalkyl or $C_3$-$C_{10}$-cycloalkoxy radical, a phenyl, phenyloxy, phenylthio, phenylsulphinyl, phenylsulphonyl or phenylamino group, whereby the phenyl moieties of the previously mentioned radicals can be substituted one or more times by hydroxyl, halogen, $C_1$-$C_6$-alkyl, $C_1$-$C_6$-alkoxy, trifluoromethyl, cyano, nitro, amino, $C_1$-$C_6$-alkylamino or di-$C_1$-$C_6$-alkylamino groups, $R_2$ a phenyl radical which can be substituted one or more times by halogen, $C_1$-$C_6$-alkyl, $C_1$-$C_6$-alkoxy and trifluoromethyl groups or, for the case that B is an alkylene group, also hydrogen, A the groups $-(CH_2)_o-$ with o = 3-10 or $-(CH_2)_p\text{-}X\text{-}(CH_2)_q-$ with p = 2-8 and q = 1-6, whereby o, p and q signify whole numbers and the sum of p and q must not be greater than 10 and X represents an oxygen or sulphur atom or the group –NH– or, for the case that $R_1$ represents a phenyl radical as given in the definition of $R_1$, A also signifies the groups $-CH_2\text{-}X\text{-}(CH_2)_q$, $-CH{=}CH\text{-}CH_2-$, $-C{\equiv}C\text{-}CH_2-$, $-C{\equiv}C\text{-}(CH_2)_p-$, $-CH{=}CH\text{-}CH_2\text{-}X\text{-}(CH_2)_q-$, $-C{\equiv}C\text{-}CH_2\text{-}X\text{-}(CH_2)_q-$, whereby p, q and X possess the above-given definition, Y signifies the group $-S(O)_n-$ or –O– with n = 0, 1 or 2 and B signifies a valency bond or the group $-(CH_2)_r-$ with r = 1-18, $-CH_2\text{-}CH{=}CH-$, $-CH_2\text{-}C{\equiv}C-$ or $-CH{=}CH-$, with the exception of the compounds 2-phenoxy-5-phenylvaleric acid, 2-phenylthio-5-phenyl-4-pentenoic acid and 2-phenylsulphonyl-5-phenyl-4-pentenoic acid, as well as their physiologically acceptable salts, esters and amides.

2. Carboxylic acid derivatives of the formula I according to claim 1 in which A signifies the group $-(CH_2)_o-$ with o = 4, 5 or 6.

3. Carboxylic acid derivatives of the formula I according to claim 1, in which $R_1$ signifies a phenyl or phenoxy radical which can possibly be substituted one or more times by halogen, $C_1$-$C_6$-alkyl, $C_1$-$C_6$-alkoxy or trifluoromethyl groups.

4. Carboxylic acid derivatives of the formula I according to claim 1, in which $R_2$ signifies a phenyl, 2-methylphenyl, 3-methylphenyl, 4-methylphenyl, 4-tert.-butylphenyl, 4-methoxy- and 3-trifluoromethylphenyl radical.

5. Carboxylic acid derivatives according to claim 1 selected from the group of the following compounds :
8-methoxy-2-(4-methylphenylsulphonyl)-octanoic acid
8-methylthio-2-(4-methylphenylsulphonyl)-octanoic acid
8-methylsulphonyl-2-(4-methylphenylsulphonyl)-octanoic acid
8-(4-chlorophenyl)-2-(3-methylphenylsulphonyl)-octanoic acid
4-[2-(4-chlorophenyl)-ethoxy]-2-(4-methylphenylsulphonyl)-butanoic acid
7-phenyl-2-[2-(4-t-butylphenyl)-ethylsulphonyl]-heptanoic acid
8-phenyl-2-(4-t-butylphenoxy)-octanoic acid
8-phenyl-2-(4-t-butylphenylthio)-octanoic acid
8-phenyl-2-(4-ethylphenylsulphonyl)-octanoic acid
8-phenyl-2-(4-propylphenylsulphonyl)-octanoic acid
8-phenyl-2-(4-t-butylphenylsulphonyl)-octanoic acid
7-(4-chlorophenyl)-2-(4-t-butylphenoxy)-heptanoic acid
7-(4-chlorophenyl)-2-(4-t-butylphenylthio)-heptanoic acid
7-(4-chlorophenyl)-2-(4-t-butylphenylsulphonyl)-heptanoic acid
8-(4-chlorophenyl)-2-(4-t-butylphenoxy)-octanoic acid
8-(4-chlorophenyl)-2-(4-t-butylphenylthio)-octanoic acid
8-(4-chlorophenyl)-2-(4-ethylphenylsulphonyl)-octanoic acid
8-(4-chlorophenyl)-2-(4-propylphenylsulphonyl)-octanoic acid
8-(4-chlorophenyl)-2-(4-t-butylphenylsulphonyl)-octanoic acid
7-(4-methoxyphenyl)-2-(4-t-butylphenoxy)-heptanoic acid

7-(4-methoxyphenyl)-2-(4-t-butylphenylthio)-heptanoic acid
7-(4-methoxyphenyl)-2-(4-t-butylphenylsulphonyl)-heptanoic acid
6-phenoxy-2-(4-t-butylphenoxy)-hexanoic acid
6-(4-chlorophenoxy)-2-(4-methylphenoxy)-hexanoic acid
6-(4-chlorophenoxy)-2-(4-t-butylphenoxy)-hexanoic acid.

6. Medicaments containing at least one compound according to claim 1-5 or the compound 2-phenylthio-5-phenyl-4-pentenoic acid or 2-phenylsulphonyl-5-phenyl-4-pentenoic acid or their physiologically acceptable salts, esters or amides, besides usual carrier and adjuvant materials.

7. Medicaments according to claim 6 with antidiabetic action.

8. Use of compounds according to claim 1-5 for the preparation of medicaments with anti-diabetic action.

**Claims for the Contracting States : ES, GR**

1. Process for the preparation of carboxylic acid derivatives of the general formula I

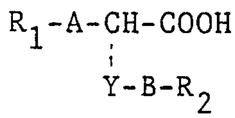

(I)

in which $R_1$ signifies a $C_1$-$C_8$-alkoxy, $C_1$-$C_8$-alkylthio, $C_1$-$C_8$-alkylsulphinyl, $C_1$-$C_8$-alkylsulphonyl or $C_1$-$C_8$-alkylamino radical, a $C_3$-$C_{10}$-cycloalkyl or $C_3$-$C_{10}$-cycloalkoxy radical, a phenyl, phenyloxy, phenylthio, phenylsulphinyl, phenylsulphonyl or phenylamino group, whereby the phenyl moieties of the previously mentioned radicals can be substituted one or more times by hydroxyl, halogen, $C_1$-$C_6$-alkyl, $C_1$-$C_6$-alkoxy, trifluoromethyl, cyano, nitro, amino, $C_1$-$C_6$-alkylamino or di-$C_1$-$C_6$-alkylamino groups, $R_2$ a phenyl radical which can be substituted one or more times by halogen, $C_1$-$C_6$-alkyl, $C_1$-$C_6$-alkoxy and trifluoromethyl groups or, for the case that B is an alkylene group, also hydrogen, A the groups $-(CH_2)_o-$ with o = 3-10 or $-(CH_2)_p-X-(CH_2)_q-$ with p = 2-8 and q = 1-6, whereby o, p and q signify whole numbers and the sum of p and q must not be greater than 10 and X represents an oxygen or sulphur atom or the group –NH– or, for the case that $R_1$ represents a phenyl radical as given in the definition of $R_1$, A also signifies the groups $-CH_2-X-(CH_2)_q-$, $-CH=CH-CH_2-$, $-C{\equiv}C-CH_2-$, $-C{\equiv}C-(CH_2)_p-$, $-CH=CH-CH_2-X-(CH_2)_q-$, $-C{\equiv}C-CH_2-X-(CH_2)_q-$, whereby p, q and X possess the above-given definition, Y signifies the group $-S(O)_n-$ or –O– with n = 0, 1 or 2 and B signifies a valency bond or the group $-(CH_2)_r-$ with r = 1-18, $-CH_2-CH=CH-$, $-CH_2-C{\equiv}C-$ or –CH=CH–, with the exception of the compounds 2-phenoxy-5-phenylvaleric acid, 2-phenylthio-5-phenyl-4-pentenoic acid and 2-phenylsulphonyl-5-phenyl-4-pentenoic acid, as well as their physiologically acceptable salts, esters and amides, characterised in that one

A) reacts a group of the general formula II

in which W signifies $-COOR_3$ or another group convertible into the carboxyl function, X a reactive residue and Z hydrogen or the group $R_1$-A–, in which $R_1$ and A possess the above-given meanings and $R_3$ signifies lower alkyl, with a group of the general formula III

$R_2$-B-YH (III)

in which $R_2$, B and Y possess the above-given meanings, to give a compound of the general formula IV

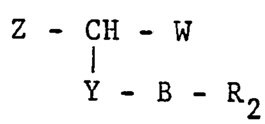

(IV)

and subsequently thereto

a) converts the group W into the free carboxyl function, a physiologically acceptable ester or an amide,
b) for the case that Y represents a sulphur atom, converts the group Y into the group SO or $SO_2$ or
c) for the case that Z signifies hydrogen, either
c1) alkylates with a compound of the general formula V

$$R_1\text{-A-X} \quad (V)$$

in which $R_1$ and A have the above-given meanings and X signifies a reactive group, or
c2) condenses with a compound of the general formula V′

$$R_1\text{-A'-CHO} \quad (V')$$

in which A′ signifies an alkylene radical shortened by the $-CH_2-$ group and $R_1$ possesses the above-given meaning, and, subsequent to the condensation, hydrogenates the resultant double bond and converts compounds IV with Z = $R_1A-$ obtained according to the above-mentioned process steps c1) and c2) in the above-given manner into the groups W or Y or
B) reacts a compound of the general formula VI

$$R_1 - A - \underset{\substack{|\\ YH}}{CH} - W \qquad (VI),$$

in which $R_1$, A, Y and W possess the above-given meanings, with a group of the general formula VII

$$R_2\text{-B-X} \quad (VII)$$

in which $R_2$, B and X possess the above-given meanings, to give a compound of the formula IV and possibly converts this in the above-given manner into the groups W and Y and subsequently, if desired, converts the compounds obtained into physiologically acceptable amides, esters or salts.

2. Process for the preparation of carboxylic acid derivatives of formula I according to claim 1, in which A signifies the group $-(CH_2)_o-$ with o = 4, 5 or 6.

3. Process for the preparation of carboxylic acid derivatives of formula I according to claim 1, in which $R_1$ signifies a phenyl or phenoxy radical which can possibly be substituted one or more times by halogen, $C_1$-$C_4$-alkyl, $C_1$-$C_8$-alkoxy and trifluoromethyl groups.

4. Process for the preparation of carboxylic acid derivatives of formula I according to claim 1, in which $R_2$ is a phenyl, 2-methylphenyl, 3-methylphenyl, 4-methylphenyl, 4-tert.-butylphenyl, 4-methoxy- or 3-trifluoromethylphenyl radical.

5. Process for the preparation of carboxylic acid derivatives according to claim 1 selected from the group of the following compounds

8-methoxy-2-(4-methylphenylsulphonyl)-octanoic acid
8-methylthio-2-(4-methylphenylsulphonyl)-octanoic acid
8-methylsulphonyl-2-(4-methylphenylsulphonyl)octanoic acid
8-(4-chlorophenyl)-2-(3-methylphenylsulphonyl)octanoic acid
4-[2-(4-chlorophenyl)-ethoxy]-2-(4-methylphenylsulphonyl)-butanoic acid
7-phenyl-2-[2-(4-t-butylphenyl)-ethylsulphonyl]heptanoic acid
8-phenyl-2-(4-t-butylphenoxy)-octanoic acid
8-phenyl-2-(4-t-butylphenylthio)-octanoic acid
8-phenyl-2-(4-ethylphenylsulphonyl)-octanoic acid
8-phenyl-2-(4-propylphenylsulphonyl)-octanoic acid
8-phenyl-2-(4-t-butylphenylsulphonyl)-octanoic acid
7-(4-chlorophenyl)-2-(4-t-butylphenoxy)-heptanoic acid
7-(4-chlorophenyl)-2-(4-t-butylphenylthio)-heptanoic acid
7-(4-chlorophenyl)-2-(4-t-butylphenylsulphonyl)heptanoic acid
8-(4-chlorophenyl)-2-(4-t-butylphenoxy)-octanoic acid
8-(4-chlorophenyl)-2-(4-t-butylphenylthio)-octanoic acid
8-(4-chlorophenyl)-2-(4-ethylphenylsulphonyl)-octanoic acid
8-(4-chlorophenyl)-2-(4-propylphenylsulphonyl)-octanoic acid

8-(4-chlorophenyl)-2-(4-t-butylphenylsulphonyl)octanoic acid
7-(4-methoxyphenyl)-2-(4-t-butylphenoxy)-heptanoic acid
7-(4-methoxyphenyl)-2-(4-t-butylphenylthio)-heptanoic acid
7-(4-methoxyphenyl)-2-(4-t-butylphenylsulphonyl)- heptanoic acid
6-phenoxy-2-(4-t-butylphenoxy)-hexanoic acid
6-(4-chlorophenoxy)-2-(4-methylptienoxy)-hexanoic acid
6-(4-chlorophenoxy)-2-(4-t-butylphenoxy)-hexanoic acid.

6. Process for the preparation of medicaments containing, as active material, at least one compound of formula I according to one of claims 1-5 or the compound 2-phenylthio-5-phenyl-4-pentenoic acid or 2-phenylsulphonyl-5-phenyl-4-pentenoic acid or their physiologically acceptable salts, esters and amides, characterised in that one works up the active materials, together with usual carrier and adjuvant materials, to give medicaments.